# EUROPÄISCHE PATENTSCHRIFT

(11) **EP 1 319 368 B1**
(45) Veröffentlichungstag und Bekanntmachung des Hinweises auf die Patenterteilung: **30.05.2007**
(21) Anmeldenummer: 01130800.4
(22) Anmeldetag: 24.12.2001
(51) Int. Cl.: A61B 6/12, A61B 19/00

(54) **Verfahren zur Bestimmung der Orientierung und Relativposition eines medizinischen Instruments**
Method for determining the orientation and relative position of a medical instrument
Méthode pour déterminer l'orientation et la position relative d'un instrument médical

(30) Priorität: 12.12.2001 DE 10161160
(43) Veröffentlichungstag der Anmeldung: 18.06.2003
(73) Patentinhaber: TECMEDIC GmbH, 45881 Gelsenkirchen (DE)
(72) Erfinder: Winkler, Wolfgang, 52477 Alsdorf (DE); Neddermeyer, Werner, 64367 Mühltal (DE); Dreckmann, Mathias, 46282 Dorsten (DE)
(74) Vertreter: Mierswa, Klaus

(56) Entgegenhaltungen:
- EP-A- 0 728 446
- WO-A-99/27839
- DE-A- 19 536 180
- US-A- 6 144 875

## Beschreibung

### Technisches Gebiet:

Die Erfindung betrifft ein Verfahren zur Bestimmung der Orientierung und Relativposition eines medizinischen Instruments, z.B. Sonde oder Injektionsnadel, gegenüber einer medizinisch relevanten Struktur im Körper eines Menschen oder Tieres zum Zweck eines medizinischen Eingriffs.

### Stand der Technik:

In der Medizintechnik ist die so genannte 3D-Navigation bekannt und wird z.B. im Bereich der HNO-Chirurgie und Neurochirurgie eingesetzt. Aufgabe der 3D-Navigation ist es hierbei, den Chirurgen über die Orientierung und Relativposition eines medizinischen Instruments, welches er bei einem medizinischen Eingriff in den Körper des Patienten einführt, gegenüber einer medizinisch relevanten Struktur, z.B. Tumor, welchem der Eingriff gilt, zu informieren, um den Chirurgen während des Eingriffs zu führen und ihm ein möglichst minimalinvasives Vorgehen zu ermöglichen. Einige entsprechende Systeme werden in der Veröffentlichung "Computergestützte 3D-Navigationssysteme", erschienen in HNO 2/2000, 48, Seiten 75-90, Springer-Verlag 2000, von A.R. Gunkel, W.F. Thumfart und W. Freysinger beschrieben. Methoden der 3D-Navigation sind in der Veröffentlichung "Grenzen der CTbasierten Computernavigation in der Wirbelsäulenchirurgie", erschienen in UNFALLCHIRURG 8/2000, 103, Seiten 696-701, Springer-Verlag 2000, von F. Gebhard, L. Kinzl und M. Arand, erläutert.

Nachteilig bei diesen bekannten Verfahren zur 3D-Navigation ist, daß die atmungsbedingte Verformung des Körpers bei der Navigation nicht berücksichtgt wird, was den Anwendungsbereich dieser Verfahren auf solche Körperbereiche einschränkt, welche atmungsbedingten Verformung praktisch nicht unterworfen sind, wie z.B. der Kopf.

Der Erfindung liegt daher die Aufgabe zu Grunde, ein Verfahren bereitzustellen, mit welchem die Bestimmung der Orientierung und der Relativposition eines medizinischen Instruments gegenüber einer medizinisch relevanten Struktur im Körper eines atmenden Menschen oder atmenden Tieres unter Berücksichtigung atmungsbedingter Verformungen des Körpers ermöglicht wird.

Diese Aufgabe wird erfindungsgemäß gelöst durch ein Verfahren zur Bestimmung der Orientierung und der Relativposition eines medizinischen Instruments gegenüber einer medizinisch relevanten Struktur, z.B. Knochen oder Tumor, im Körper eines atmenden Menschen oder atmenden Tieres, dessen Oberfläche einen Oberflächenabschnitt umfaßt, welcher sich aufgrund der Atmung gegenüber der Struktur zumindest zeitweise bewegt, zum Zweck eines medizinischen Eingriffs, wobei das medizinische Instrument mit mindestens zwei voneinander beabstandeten, an demselben starr angeordneten Markierungen versehen ist, deren Relativposition gegenüber dem medizinischen Instrument jeweils bekannt ist, umfassend folgende Schritte:
a) in wenigstens einer ersten Phase eines Atemzyklusses des Menschen oder Tieres wird mindestens ein die Struktur enthaltender Teil des Körpers einer Kernspin- oder Computer-Tomographie unterzogen, mittels welcher die Struktur sowie eine den Oberflächenabschnitt aufpannende Vielzahl von Oberflächenpunkten erfaßt und die in der ersten Phase vorliegende gegenseitige Relativposition der Oberflächenpunkte sowie die Relativposition der Struktur gegenüber mindestens einem der Oberflächenpunkte bestimmt werden,
b) auf dem Oberflächenabschnitt wird ein Satz von mindestens drei nicht kollinearen Referenzmarken, welche eine ebene oder nicht ebene Referenzfläche aufspannen, die an der atmungsbedingten Bewegung des Oberflächenabschnitts teilnimmt, ausgewählt, wobei der Ort jeder Referenzmarke
   - in der ersten Phase jedes Atemzyklusses durch je einen der Referenzmarke ein-eindeutig zugeordneten ersten Aufpunkt, welche eine ebene oder nicht ebene erste Aufpunkt-Referenzfläche aufspannen,
   - in einer zweiten Phase jedes Atemzyklusses durch je einen der Referenzmarke ein-eindeutig zugeordneten zweiten Aufpunkt, welche eine ebene oder nicht ebene zweite Aufpunkt-Referenzfläche aufspannen, und
   - in einer dritten Phase jedes Atemzyklusses durch je einen der Referenzmarke ein-eindeutig zugeordneten dritten Aufpunkt, welche eine ebene oder nicht ebene zweite Aufpunkt-Referenzfläche aufspannen,
   gegeben ist,
c) mittels mindestens zwei voneinander beabstandeten ersten optischen Meßeinrichtungen wird die Relativposition von mindestens einem der ersten Aufpunkte gegenüber mindestens einem der zweiten Aufpunkte stereographisch oder trigonometrisch oder durch Bündelausgleich bestimmt,
d) mittels mindestens zwei voneinander beabstandeten zweiten optischen Meßeinrichtungen werden die gegenseitigen Relativpositionen von mindestens dreien der zweiten Aufpunkte stereographisch oder trigonometrisch oder durch Bündelausgleich bestimmt,
e) die Relativposition von mindestens einem der zweiten Aufpunkte gegenüber mindestens einem der Oberflächenpunkte wird aus dem Ergebnis der Schritts c) und durch Ermittlung der Position der ersten oder der zweiten Aufpunkt-Referenzfläche auf dem Oberflächenabschnitt durch Matching zwischen der Orientierung und Gestalt der ersten bzw. zweiten Aufpunkt-Referenzfläche und der Orientierung und der Gestalt des Oberflächenabschnitts bestimmt,
f) mittels mindestens zwei voneinander beabstandeten dritten optischen Meßeinrichtungen wird die gegenseitige Relativposition von mindestens einem der dritten Aufpunkte gegenüber mindestens einem der ersten oder zweiten Aufpunkte stereographisch oder trigonometrisch oder durch Bündelausgleich bestimmt,
g) mittels mindestens zwei voneinander beabstandeten vierten optischen Meßeinrichtungen wird die Relativposition von mindestens zwei der Markierungen des medizinischen Instruments gegenüber jeweils mindestens einem der dritten Aufpunkte stereographisch oder trigonometrisch oder durch Bündelausgleich bestimmt, und
h) aus den so gewonnenen Ergebnissen werden für die dritte Phase des Atemzyklusses die Orientierung und die Relativposition des medizinischen Instruments gegenüber der Struktur bestimmt.

Die medizinisch relevante Struktur kann z.B. ein Knochen, ein Gelenk, ein Tumor, ein eingedrungener Fremdkörper oder ein Teil eines inneren Organs sein. Das medizinische Instrument kann z.B. eine Sonde oder eine Injektionsnadel sein. Mit Hilfe des erfindungsgemäßen Verfahrens wird vorteilhafterweise der Einfluß der Atmung auf die Relativposition des medizinischen Instruments gegenüber der Struktur berücksichtigt.

Alle genannten Relativpositionen beziehen sich auf alle drei Raumrichtungen, d.h. es werden dreidimensionale Informationen ermittelt, wobei die Ermittlung bzw. Berechnung der Relativpositionen bevorzugt, jedoch nicht notwendigerweise in kartesischen Koordinaten erfolgt.

Selbstverständlich wird für jeden Verfahrensschritt eine endliche Zeit benötigt. Mit erster, zweiter und dritter Phase sind daher jeweils Phasenbereiche endlicher Breite gemeint, innerhalb welcher die entsprechenden Verfahrensschritte ausführbar sind. Diese Breiten der Phasenbereiche werden bevorzugt sehr klein im Vergleich zur Dauer eines Atemzyklus gehalten.

Gemäß einer bevorzugten Variante der Erfindung fallen die erste und die zweite Phase bezüglich ihrer Lage im Atemzyklus zusammen, so daß die ersten Aufpunkte mit den zweiten Aufpunkten identisch sind, wobei der Verfahrensschritt c) entfällt. Gemäß einer anderen Variante fallen die erste und die dritte Phase bezüglich ihrer Lage im Atemzyklus zusammen, so daß die ersten Aufpunkte mit den dritten Aufpunkten identisch sind, und der Verfahrensschritt f) entfällt.

Gemäß einer Kombination dieser beiden Varianten fallen daher die erste, die zweite und die dritte Phase bezüglich ihrer Lage im Atemzyklus zusammen, so daß die ersten Aufpunkte mit den zweiten und den dritten Aufpunkten identisch sind und die Verfahrensschritte c) und f) entfallen.

Die ersten optischen Meßeinrichtungen befinden sich bevorzugt innerhalb des Kernspin- oder Computer-Tomographen und können auf herkömmliche Weise gegeneinander und gegen den Kernspin- oder Computer-Tomographen kalibriert, d.h. bezüglich ihrer Lage eingemessen werden. Beispielsweise kann zur Kalibrierung an einer definierten Stelle in dem Kernspin- oder Computer-Tomographen ein mit Markierungen versehendes Kalibriergestell aufgestellt werden, welches dazu dient, Zielmarken zur Bestimmung der gegenseitigen Relativlage der ersten optischen Meßeinrichtungen und der Relativlage derselben gegenüber dem Kernspin- oder Computer-Tomographen bereitzustellen. Auch die zweiten, dritten und vierten optischen Meßeinrichtungenjeweils können in herkömmlicher Weise gegeneinander eingemessen werden.

Die ersten, zweiten, dritten und vierten optischen Meßeinrichtungen können z.B. jeweils Videokameras sein. Bevorzugt ist die Anzahl der ersten, zweiten, dritten und vierten optischen Meßeinrichtungen jeweils gleich vier.

Die ersten und/oder zweiten und/oder dritten und/oder vierten optischen Meßeinrichtungen können identisch sein. Gemäß einer bevorzugten Variante der Erfindung sind die zweiten, dritten und vierten optischen Meßeinrichtungen identisch und befinden sich außerhalb des Kernspin- oder Computer-Tomographen.

Die erste Phase und die dritte Phase können z.B. so gewählt sein, daß im Atemzyklus des Menschen oder Tieres die erste Phase jeweils dem Übergang vom Einatmen zum Ausatmen und die dritte Phase jeweils dem Übergang vom Ausatmen zum Einatmen entspricht.

Die Referenzmarken können besonders vorteilhaft auf einer Trägerfläche, z.B. Pflaster, markiert sein, welche auf der Oberfläche des Körpers lösbar fixiert, z.B. aufgeklebt wird.

Gemäß einer bevorzugten Variante der Erfindung wird der Verfahrensschritt a) für eine Mehrzahl oder Vielzahl verschiedener, in ihrer Phasenlage bezüglich des Atemzyklus voneinander beabstandeter erster Phasen wiederholt, so daß für jede der ersten Phasen ein eigener, zu dieser ersten Phase gehörender Satz von gegenseitigen Relativpositionen der Oberflächenpunkte sowie eine zu dieser ersten Phase gehörende Relativposition der Struktur gegenüber mindestens einem der Oberflächenpunkte bestimmt werden.

Ebenso wird gemäß einer bevorzugten Variante der Verfahrensschritt d) für eine Mehrzahl oder Vielzahl verschiedener, in ihrer Phasenlage bezüglich des Atemzyklus voneinander beabstandeter zweiter Phasen wiederholt, so daß für jede der zweiten Phasen ein eigener, zu dieser zweiten Phase gehörender Satz von gegenseitigen Relativpositionen der zweiten Aufpunkte bestimmt wird.

Dabei können die Anzahl der ersten Phasen und die Anzahl der zweiten Phasen vorteilhaft miteinander identisch sein, so daß jeder der ersten Phasen genau eine der zweiten Phasen zugeordnet ist, und die Phasenlage jeder der ersten Phasen mit der Phasenlage der ihr zugeordneten zweiten Phase übereinstimmt.

Gemäß einer Verfeinerung des erfindungsgemäßen Verfahren kann jede Durchführung des Verfahrensschrittes a) mit einer Durchführung des Verfahrensschrittes d) zeitlich zusammenfallen, so daß die Durchführung der Verfahrensschritte a) mit der Durchführung des Verfahrensschrittes d) jeweils synchron erfolgt.

Die Durchführung des Verfahrensschrittes a) braucht für die verschiedenen ersten Phasen nicht notwendigerweise innerhalb desselben Atemzyklus zu erfolgen; vielmehr kann die Durchführung des Verfahrensschrittes a) für eine der ersten Phasen in einem ersten Atemzyklus, für eine andere der ersten Phasen in einem zweiten Atemzyklus, für wieder eine andere der ersten Phasen in einem dritten Atemzyklus usw. erfolgen. Ebenso braucht die Durchführung des Verfahrensschrittes d) für die verschiedenen zweiten Phasen nicht notwendigerweise innerhalb eines einzigen Atemzyklus zu erfolgen; vielmehr kann die Durchführung des Verfahrensschrittes b) für eine der zweiten Phasen z.B. in einem vierten Atemzyklus, für eine andere der zweiten Phasen in einem fünften Atemzyklus, für wieder eine andere der zweiten Phasen in einem sechsten Atemzyklus usw. erfolgen.

Gemäß einer bevorzugten Variante erfolgen jedoch alle Wiederholungen des Verfahrensschritts a) und des Verfahrensschrittes d) innerhalb eines einzigen, nämlich eines ersten Atemzyklusses. Wenn z.B. der Atemzyklus eine Dauer von X Sekunden hat, kann diese Dauer z.B. in Y gleiche Teile zerlegt werden, so daß jeder Teil X/Y Sekunden dauert., und der Verfahrensschritt a) kann während des ersten Atemzyklus in Zeitabständen von X/Y Sekunden insgesamt Y mal wiederholt werden, so daß Y verschiedene erste Phasen vorliegen, welche gleichmäßig über den Atemzyklus verteilt sind.

Gemäß einer bevorzugten Variante der Erfindung werden die Verfahrensschritte f) bis h) für eine Mehrzahl oder Vielzahl verschiedener, in ihrer Phasenlage bezüglich des Atemzyklus voneinander beabstandeter dritter Phasen wiederholt, so daß für jede der dritten Phasen die Orientierung und die Relativposition des medizinischen Instruments gegenüber der Struktur neu bestimmt werden.

Gemäß einer bevorzugten Variante der Erfindung sind die Anzahl der zweiten Phasen und die Anzahl der dritten Phasen miteinander identisch, so daß jeder der dritten Phasen eine der zweiten Phasen eindeutig zugeordnet ist, und die Phasenlage jeder der dritten Phasen mit der Phasenlage der ihr zugeordneten zweite Phasen übereinstimmt, wobei für jede der dritten Phasen die Orientierung und die Relativposition des medizinischen Instruments gegenüber der Struktur unter Verwendung
- desjenigen Satzes von gegenseitigen Relativpositionen der zweiten Aufpunkte, welcher zu der dieser dritten Phase zugeordneten zweiten Phase gehört,
- desjenigen Satzes von gegenseitigen Relativpositionen der Oberflächenpunkte, welcher zu der dieser zweiten Phase zugeordneten ersten Phase gehört, und
- derjenigen Relativposition der Struktur gegenüber mindestens einem der Oberflächenpunkte, welche zu der dieser zweiten Phase zugeordneten ersten Phase gehört,
neu bestimmt wird.

Auf diese Weise werden bei der Bestimmung der Orientierung und Relativposition des medizinischen Instruments gegenüber der Struktur nicht nur die atmungsbedingte Hebung und Senkung der Referenzfläche berücksichtigt, sondern sehr vorteilhaft auch alle atmungsbedingten Verschiebungen der Struktur innerhalb des Körpers sowie alle atmungsbedingten Verzerrungen, Verformungen und Orientierungsänderungen der Referenzfläche.

Die Verfahrensschritte f) bis h) werden bevorzugt erst nach Ende des ersten Atemzyklusses und bevorzugt außerhalb des Kernspin- oder Computer-Tomographen durchgeführt.

Insbesondere können die Verfahrensschritte f) bis h) über eine Mehrzahl von Atemzyklen hinweg fortlaufend wiederholt werden. Die Wiederholung der Verfahrensschritte f) bis h) kann so lange fortgesetzt werden wie es für den medizinischen Eingriff erforderlich ist.

Gemäß einer weiteren Variante der Erfindung können die Orientierung und die Relativposition des medizinischen Instruments gegenüber der Struktur nicht nur für solche Zeitpunkt ermittelt werden, welche auf eine der dritten Phasen fallen, sondern für beliebige Zeitpunkte bzw. Phasen. Gemäß dieser Variante wird die Orientierung bzw. die Relativposition des medizinischen Instruments gegenüber der Struktur für eine beliebige vierte Phase des Atemzyklusses gewonnen werden, indem zwischen den für zwei verschiedene dritten Phasen ermittelten Orientierungen des medizinischen Instruments bzw. zwischen den für zwei verschiedene dritten Phasen ermittelten Relativposition des medizinischen Instruments gegenüber der Struktur linear oder nichtlinear interpoliert wird. Vorzugsweise werden dabei für die Interpolation diejenigen beiden dritten Phasen herangezogen, welche der vierten Phase benachbart sind.

Insbesondere kann die jeweils zuletzt bestimmte Orientierung und Relativposition des medizinischen Instruments gegenüber der Struktur auf mindestens einem Monitor graphisch dargestellt werden, um dem den Eingriff vornehmenden Chirurgen als Navigationshilfe zu dienen. Gemäß einer Verfeinerung dieser Variante erfolgt die Darstellung in Projektion auf verschiedene Ebenen, deren Normalen verschiedenen Blickrichtungen entsprechen, auf mehreren Monitoren zugleich. Gemäß einer weiteren Variante werden die Struktur und das medizinische Instrument auf einem Monitor in Stereo-Darstellung gezeigt, wobei der Chirurg eine Stereobrille trägt. Auf dem Monitor bzw. den Monitoren kann zusätzlich die jeweils zuletzt bestimmte Relativposition der Referenzmarken gegenüber der Struktur in Echtzeit dargestellt werden, um die Navigation weiter zu erleichtern.
Die Darstellung auf dem Monitor bzw. den Monitoren kann dabei jeweils in Echtzeit erfolgen. Bei hinreichend dichter Wahl der ersten bzw. zweiten bzw. dritten Phasen kann auf dem Monitor bzw. den Monitoren eine filmähnliche Bildfolge dargestellt werden. Dabei kann insbesondere die Information über die Relativposition der Referenzmarken untereinander und gegenüber der Struktur aus den innerhalb des Kernspin- oder Computertomographen während des ersten Atemzyklus für eine Vielzahl von ersten Phasen jeweils durchgeführten Verfahrenschritten a) stammen, sich also für alle folgenden Atemzyklen stets auf den ersten Atemzyklus beziehen, während die Orientierung und die Relativposition des medizinischen Instruments gegenüber den Refrenzmarken laufend "on-line" neu bestimmt wird. Die Darstellung auf dem Monitor setzt sich in dieser Variante der Erfindung demnach zusammen aus Informationen, welche aus dem ersten Atemzyklus stammen und dort jeweils nur einmal gewonnen und gespeichert wurden, und solchen, welche in späteren Atemzyklen fortlaufend gewonnen werden.

Insbesondere kann zwischen dem Verfahrensschritt a) und den Verfahrensschritten g) und h) eine beliebige Zeitspanne liegen. Änderungen der Atemfrequenz, welche eine derartige Überlagerung von Informationen stören würden, können dabei erfaßt und rechnerisch durch eine entsprechende zeitliche Anpassung, d.h. rechnerische Dehnung oder Komprimierung des ersten Atemzyklusses berücksichtigt werden.

Die Markierungen brauchen nicht notwendigerweise unmittelbar an dem medizinischen Instrument angeordnet zu sein. Vielmehr können sie auch starr an einem Trägerkörper angeordnet sein, welcher seinerseits mit dem medizinischen Instrument lösbar, jedoch starr verbunden ist, wobei im Falle eines Wechsels des medizinischen Instruments ein- und derselbe Trägerkörper nacheinander an verschiedenen medizinischen Instrumenten zum Einsatz kommen kann.

Mit Hilfe des erfindungsgemäßen Verfahrens kann der Einfluß der Atmung auf die Relativposition des medizinischen Instruments gegenüber der Struktur für unbegrenzte Zeit fortlaufend berücksichtigt und graphisch dargestellt werden, wobei der Körper vorteilhafterweise nur für kurze Zeit einer Kernspin- oder Computer-Tomographie ausgesetzt zu werden braucht.

Kurzbeschreibung der Zeichnung, in der schematisch zeigen:
- Fig.: 1 einen Körper, welcher eine medizinisch relevante Struktur enthält, innerhalb eines Kernspin- oder Computer-Tomographen in Querschnittsdarstellung,
- Fig. 2: einen Oberflächenabschnitt des Körpers von Fig. 1 mit darauf angebrachten Referenzmarken in Draufsicht, wobei die Referenzmarken eine Referenzfläche aufspannen,
- Fig. 3: die atmungsbedingte Verschiebung der Referenzfläche von Fig. 2,
- Fig. 4: die Bestimmung der gegenseitigen Relativpositionen der Referenzmarken von Fig. 2 mittels optischer Meßeinrichtungen,
- Fig. 5: ein teilweise in den Körper von Fig. 1 eingeführtes medizinisches Instrument, dessen Orientierung und Relativposition bezüglich der medizinisch relevanten Struktur von Fig. 1 bestimmt wird,
- Fig.6: eine perspektivische Darstellung des Kernspin- oder Computer-Tomographen von Fig. 1 mit einem darin angeordneten Kalibriergestell,
- Fig. 7: einen Trägerkörper, welcher an einem medizinischen Instrument starr angeordnet ist, und
- Fig. 8: eine perspektivische Darstellung des Kalibriergestells von Fig. 6.

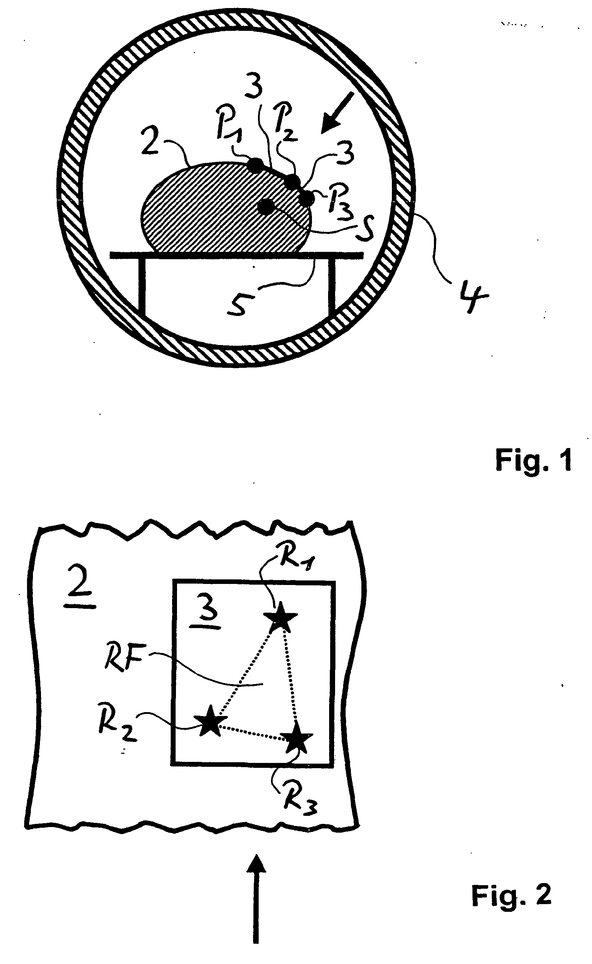

Die Fig. 1 bis 5 geben einen schematischen Überblick über den Ablauf einer bevorzugten Variante des erfindungsgemäßen Verfahrens zur Bestimmung der Orientierung und der Relativposition eines medizinischen Instruments 1 gegenüber einer medizinisch relevanten Struktur S im Körper 2 eines atmenden Menschen oder atmenden Tieres, dessen Oberfläche einen Oberflächenabschnitt 3 umfaßt, welcher sich aufgrund der Atmung gegenüber der Struktur S zumindest zeitweise bewegt. Die Struktur S kann z.B. ein Knochen, ein Tumor oder ein eingedrungener Fremdkörper sein. Das medizinische Instrument 1 dient zum Zweck eines medizinischen Eingriffs, beispielsweise um den Fremdkörper S zu entfernen, und ist mit vier voneinander beabstandeten, an demselben starr angeordneten Markierungen M1,M2,M3,M4 versehen, deren Relativposition gegenüber dem medizinischen Instrument 1 jeweils bekannt ist.

Zu einem ersten Zeitpunkt t1 (Fig. 1), welcher einer ersten Phase ϕ1 des Atemzyklusses des Menschen oder Tieres entspricht, wird ein die Struktur S enthaltender Teil des Körpers 2 einer Kernspin- oder Computer-Tomographie unterzogen. Hierzu wird der Körper 2 in einen Kernspin- oder Computer-Tomographen 4 verbracht und darin auf einem Tisch 5 gelagert. Diese Situation ist in Fig. 1 in Querschnittsdarstellung gezeigt, wobei der Oberflächenabschnitt 3 in Fig. 1 von der Kante her betrachtet wird. Mittels der Kernspin- oder Computer-Tomographie wird die Struktur S erfaßt. Ferner wird durch die Kernspin- oder Computer-Tomographie auch eine Vielzahl von Oberflächenpunkten erfaßt, die den Oberflächenabschnitt 3 aufpannen. Von der Vielzahl von Oberflächenpunkten sind in Fig. 1 aus Gründen der Anschaulichkeit nur drei Oberflächenpunkte P1,P2,P3 eingezeichnet.

Mit Hilfe der Kernspin- oder Computer-Tomographie werden die jeweils zum ersten Zeitpunkt t1 vorliegende gegenseitige Relativposition der Oberflächenpunkte P1,P2,P3 sowie außerdem die Relativposition der Struktur S gegenüber mindestens einem der Oberflächenpunkte P1,P2,P3 bestimmt; im vorliegenden Beispiel sei dies der Oberflächenpunkt P1.

Auf dem Oberflächenabschnitt 3 wird ein Satz von mindestens drei nicht kollinearen Referenzmarken R1,R2,R3 ausgewählt. Als Referenzmarken können z.B. solche gewählt werden, welche sich auf natürliche Weise von der umgebenden Oberfläche abheben und somit leicht identifizierbar sind, z.B. Leberflecken oder Male auf der Haut eines Menschen. In einer bevorzugten Variante der Erfindung werden die ausgewählten Referenzmarken R1,R2,R3 jedoch farblich gekennzeichnet, um sie gegenüber der umgebenden Oberfläche optisch abzuheben.

Fig. 2 zeigt eine Draufsicht auf den Oberflächenabschnitt 3 mit drei ausgewählten Referenzmarken R1,R2,R3, welche zur Unterscheidung von den Oberflächenpunkten P1,P2,P3 (Fig. 1), sowie um zu symbolisieren, daß es sich um Marken, nicht um mathematisch gedachte, unsichtbare Punkte handelt, sternförmig dargestellt sind. Die Blickrichtung von Fig. 2 entspricht dabei der Richtung des Pfeils von Fig. 1. Die Referenzmarken R1,R2,R3 spannen eine Referenzfläche RF auf, welche an der atmungsbedingten Bewegung des Oberflächenabschnitts 3 teilnimmt.

Die Referenzfläche RF stellt eine Annäherung an einen bestimmten Ausschnitt des Oberflächenabschnitt dar. Diese Näherung ist umso besser, je größer die Zahl der Referenzmarken ist. Im Fall von nur drei ausgewählten Referenzmarken R1,R2,R3 ist die Referenzfläche RF selbstverständlich eben und stellt einen durch eine Dreieckfläche angenäherten Ausschnitt des Oberflächenabschnitts 3 dar, welcher selbstverständlich auch im Bereich der Dreiecksfläche nicht eben zu sein braucht. Gemäß einer bevorzugten Variante der Erfindung werden 10 bis 12 Referenzmarken so ausgewählt, daß die Referenzfläche eine gute Näherung eines solchen Ausschnitts aus dem Oberflächenabschnitt 3 darstellt, welcher anhand seiner Oberflächengestalt (Orographie) und seiner Orientierung im Raum eindeutig identifizierbar ist. Die Oberflächenpunkte P1,P2,P3 von Fig. 1 sind in Fig. 2 nicht dargestellt.

Aufgrund der Atmung findet zumindest zeitweise eine Bewegung der Referenzfläche RF gegenüber der Struktur S statt. Insbesondere kann sich die Referenzfläche RF beim Einatmen heben und beim Ausatmen senken. In der ersten Phase ϕ1 des Atemzyklusses ist der Ort jeder Referenzmarke R1,R2,R3 gegeben durch je einen der Referenzmarke R1 bzw. R2 bzw. R3 ein-eindeutig zugeordneten ersten Aufpunkt R1' bzw. R2' bzw. R3'. Die ersten Aufpunkte R1',R2',R3' spannen eine erste Aufpunkt-Referenzfläche RF' auf. D.h., in der ersten Phase ϕ1 des Atemzyklusses nehmen die Referenzmarken R1 bzw. R2 bzw. R3 die Orte der ersten Aufpunkte R1' bzw. R2' bzw. R3' ein, und die Referenzfläche RF geht hierbei über in die erste Aufpunkt-Referenzfläche RF'.

In völliger Analogie hierzu ist in einer zweiten Phase ϕ2 des Atemzyklusses der Ort jeder Referenzmarke R1,R2,R3 gegeben durch je einen der Referenzmarke R1 bzw. R2 bzw. R3 ein-eindeutig zugeordneten zweiten Aufpunkt R1" bzw. R2" bzw. R3". Die zweiten Aufpunkte R1",R2",R3" spannen eine zweite Aufpunkt-Referenzfläche RF" auf. D.h., in der zweiten Phase ϕ2 des Atemzyklusses nehmen die Referenzmarken R1 bzw. R2 bzw. R3 die Orte der zweiten Aufpunkte R1" bzw. R2" bzw. R3" ein, und die Referenzfläche RF geht hierbei über in die zweite Aufpunkt-Referenzfläche RF". In wiederum völliger Analogie hierzu ist in einer drtiten Phase ϕ3 des Atemzyklusses der Ort jeder Referenzmarke R1,R2,R3 gegeben durch je einen der Referenzmarke R1 bzw. R2 bzw. R3 ein-eindeutig zugeordneten dritten Aufpunkt R1''' bzw. R2''' bzw. R3"'. Die dritten Aufpunkte R1''',R2''',R3''' spannen eine dritte Aufpunkt-Referenzfläche RF''' auf. D.h., in der dritten Phase ϕ3 des Atemzyklusses nehmen die Referenzmarken R1 bzw. R2 bzw. R3 die Orte der dritten Aufpunkte R1''' bzw. R2''' bzw. R3" ein, und die Referenzfläche RF geht hierbei über in die dritte Aufpunkt-Referenzfläche RF'''.

Die erste Phase ϕ1 kann z.B. so gewählt sein, daß sie dem Umkehrpunkt vom Ausatmen zum Einatmen entspricht, so daß die erste Aufpunkt-Referenzfläche RF' die tiefste Absenkung der Referenzfläche RF kennzeichnet. Ebenso kann die dritte Phase ϕ3 so gewählt sein, daß sie dem Umkehrpunkt vom Einatmen zum Ausatmen entspricht, so daß die dritte Aufpunkt-Referenzfläche RF''' die höchste Hebung der Referenzfläche RF kennzeichnet. Hierbei sind die erste und die dritte Aufpunkt-Referenzfläche RF',RF''' in der Regel nicht deckungsgleich, da sich der Oberflächenabschnitt während des Atemzyklus in Abhängigkeit von der Phase vergrößern, verkleinern und verzerren kann. Im vorliegenden Beispiel wird die dritte Aufpunkt-Referenzfläche RF''' größer sein und in der Regel auch eine andere Form aufweisen als die erste Aufpunkt-Referenzfläche RF'.

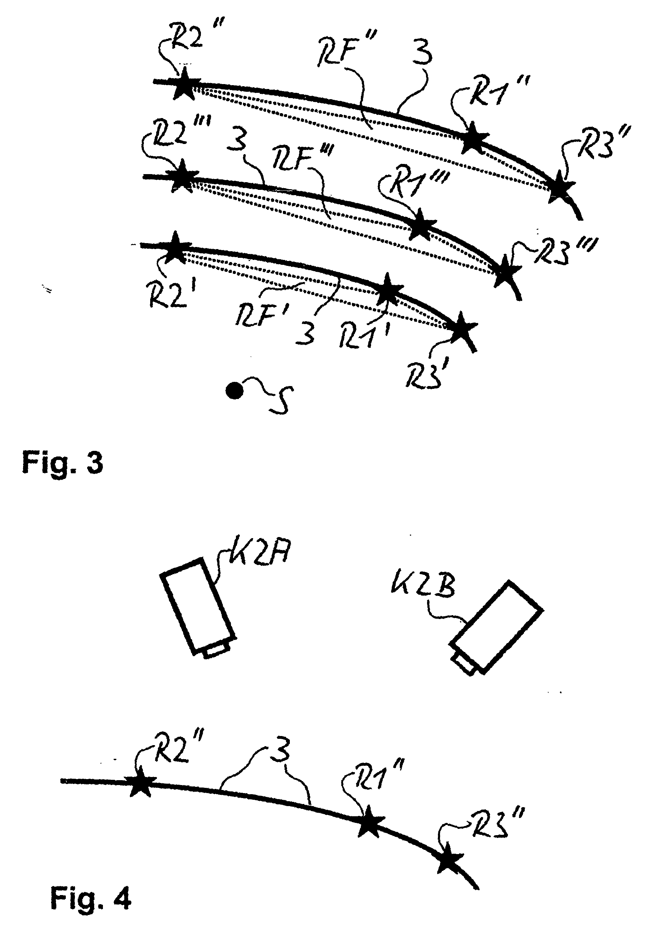

Fig. 3 zeigt schematisch die erste, zweite und dritte Aufpunkt-Referenzfläche RF',RF",RF''' mit den Aufpunkten R1',R2',R3' bzw. R1'',R2'',R3'' bzw. R1''',R2''',R3''', wobei die Blickrichtung parallel zu derjenigen von Fig. 1 ist, so daß die Aufpunkt-Referenzflächen RF',RF'',RF''' von ihren Kanten her zu sehen sind. Die erste Phase ϕ1 ist in Fig. 3 so gewählt, daß sie dem Umkehrpunkt vom Ausatmen zum Einatmen entspricht; die dritte Phase ϕ3 ist in Fig. 3 so gewählt, daß sie dem Umkehrpunkt vom Einatmen zum Ausatmen entspricht. Der gegenseitige Abstand der Aufpunkt-Referenzflächen sowie deren Größenunterschied ist in Fig. 3 stark übertrieben dargestellt.

Mittels mindestens zwei, vorzugsweise vier voneinander beabstandeten ersten optischen Meßeinrichtungen, welche vorzugsweise Video-Kameras K1A,K1B sind, wird in einem weiteren Verfahrensschritt die Relativposition von mindestens einem der ersten Aufpunkte R1',R2',R3' gegenüber mindestens einem der zweiten Aufpunkte R1",R2",R3" stereographisch oder trigonometrisch oder durch Bündelausgleich bestimmt. Dieser Verfahrensschritt kann entfallen, wenn die erste und die zweite Phase ϕ1,ϕ2 zusammenfallen, was gemäß einer bevorzugten Variante der Erfindung der Fall ist.

Mittels mindestens zwei voneinander beabstandeten zweiten optischen Meßeinrichtungen, welche vorzugsweise Video-Kameras K2A,K2B sind (Fig. 4), werden die gegenseitigen Relativpositionen von mindestens dreien der zweiten Aufpunkte R1'',R2'',R3'', bevorzugt von allen zweiten Aufpunkten, stereographisch oder trigonometrisch oder durch Bündelausgleich bestimmt, so daß die Orientierung und Gestalt der zweiten Aufpunkt-Referenzfläche RF" bekannt ist. Gemäß einer bevorzugten Variante der Erfindung werden zur Bestimmung dieser Relativpositionen vier voneinander beabstandete zweite optische Meßeinrichtungen, z.B. Video-Kameras, verwendet. Die ersten und die zweiten optischen Meßeinrichtungen sind gemäß einer Variante der Erfindung miteinander identisch.

Die Relativposition von mindestens einem der zweiten Aufpunkte R1'',R2'',R3'' gegenüber mindestens einem der Oberflächenpunkte P1,P2,P3 wird in einem weiteren Verfahrensschritt aus der oben ermittelten Relativposition von mindestens einem der ersten Aufpunkte R1',R2',R3' gegenüber mindestens einem der zweiten Aufpunkte R1",R2",R3" und durch Ermittlung der Position der ersten oder der zweiten Aufpunkt-Referenzfläche RF',RF" auf dem Oberflächenabschnitt 3 durch Matching zwischen der Orientierung und Gestalt der ersten bzw. zweiten Aufpunkt-Referenzfläche RF',RF" und der Orientierung und der Gestalt des Oberflächenabschnitts 3 bestimmt.

"Matching" ist ein im Stand der Technik bekanntes EDV-Verfahren zum Vergleich der Oberflächengestalt (Orographie) und Orientierung von zwei Flächenstücken; auf diese Weise kann ein Flächenstück einem bestimmten Abschnitt einer größeren Fläche zugeordnet und mit diesem identifiziert werden, d.h. es kann festgestellt werden, welchem Abschnitt der größeren Fläche das Flächenstück entweder vollständig oder, bei fehlender völliger Übereinstimmung, mit dem höchsten Maß an Übereinstimmung entspricht. Dadurch kann das Flächenstück auf der größeren Fläche lokalisiert werden.

Im Rahmen der Erfindung wird ein derartiges Matching dazu verwendet, die zweite Aufpunkt-Referenzfläche RF" mit einem bestimmten Ausschnitt des Oberflächenabschnitts 3 zu identifizieren oder diesem zuzuordnen, d.h. die zweite Aufpunkt-Referenzfläche RF" auf dem Oberflächenabschnitt 3 zu lokalisieren. Hierdurch sowie aus der oben ermittelten Relativposition von mindestens einem der ersten Aufpunkte R1',R2',R3' gegenüber mindestens einem der zweiten Aufpunkte R1'',R2'',R3'' ist die Relativposition von mindestens einem der zweiten Aufpunkte R1'',R2'',R3'' gegenüber mindestens einem der Oberflächenpunkte P1,P2,P3 ermittelbar.

In einem weiteren Verfahrensschritt wird mittels mindestens zwei, vorzugsweise vier voneinander beabstandeten dritten optischen Meßeinrichtungen K3A,K3B die gegenseitige Relativposition von mindestens einem der dritten Aufpunkte R"',R2"',R3"' gegenüber mindestens einem der ersten oder zweiten Aufpunkte R1',R2',R3', R1'',R2'',R3'' stereographisch oder trigonometrisch oder durch Bündelausgleich bestimmt. Die zweiten und die dritten optischen Meßeinrichtungen sind gemäß einer Variante der Erfindung miteinander identisch.

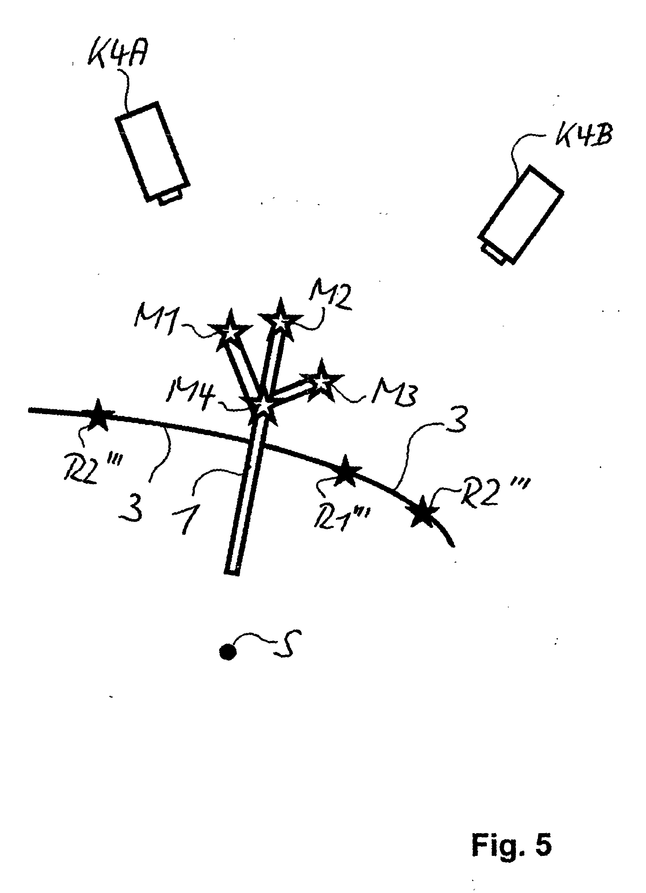

Fig. 5 dient zur Veranschaulichung eines weiteren Verfahrensschritts. Das medizinische Instrument 1 ist im Beispiel von Fig. 5 zum Zweck eines medizinischen Eingriffes teilweise in den Körper eingeführt, und trägt vier Markierungen M1,M2,M3,M4, welche starr an dem medizinischen Instrument angeordnet sind und sich außerhalb des Körpers befinden. Die Markierungen M1,M2,M3,M4 sind in Fig. 5 sternförmig dargestellt, um zu symbolisieren, daß es sich um Marken, nicht um mathematische, unsichtbare Punkte handelt.

Mittels mindestens zwei, bevorzugt vier voneinander beabstandeten vierten optischen Meßeinrichtungen K4A,K4B wird in diesem Verfahrensschritt die Relativposition von mindestens zwei der Markierungen M1,M2,M3,M4 des medizinischen Instruments 1 gegenüber jeweils mindestens einem der dritten Aufpunkte R1''',R2''',R3''' stereographisch oder trigonometrisch oder durch Bündelausgleich bestimmt. Hieraus ergibt sich die Orientierung und die Relativposition des medizinischen Instruments 1 gegenüber wenigstens einem der dritten Aufpunkte R1'''R2''',R3''', dessen Relativposition gegenüber der Struktur S aufgrund der obigen Verfahrensschritte bekannt oder ermittelbar ist.

Aus den so gewonnenen Ergebnissen werden für die dritte Phase ϕ3 des Atemzyklusses die Orientierung und die Relativposition des medizinischen Instruments 1 gegenüber der Struktur S bestimmt. Die dritten und die vierten optischen Meßeinrichtungen sind gemäß einer Variante der Erfindung miteinander identisch.

Ein Ziel des erfindungsgemäßen Verfahrens ist es, eine minimalinvasive Chirurgie in einem dem Chirurgen gewohnten Umfeld zu ermöglichen. Dies wird gemäß einer bevorzugten Variante der Erfindung durch ein zweistufiges Verfahren möglich. In der ersten Stufe werden in einem MR bzw. CT (Spiralscan) dreidimensionale Informationen der Patienten - speziell der Region des minimalinvasivem Eingriffes - generiert. Gleichzeitig erfolgt eine berührungslose Abtastung (Vermessung) der Oberfläche des Patienten. In der zweiten Stufe befindet sich der Patient außerhalb der Modalität in einer konventionellen Operationsumgebung. Durch eine wiederum berührungslose Abtastung (Vermessung) der Patientenoberfläche wird gemäß dieser Variante der Erfindung mit Hilfe computergestützter Verfahren der Zusammenhang zwischen Oberfläche und den in der Modalität 4 (Tomograph) gewonnenen 3D-Daten für jeden Zeitpunkt der Aufnahme hergestellt. Der Operateur erhält somit eine der aktuellen Lage des Patienten entsprechende Schnittbildinformation visualisiert und kann frei von Beschränkungen durch Bildgenerierungssysteme agieren. Über ein Navigationssystem kann die Lage der Schnittbilder eingestellt und die Position der Operationswerkzeuge 1 dargestellt werden. Dazu wird ein speziell markiertes Navigationswerkzeug verwendet.

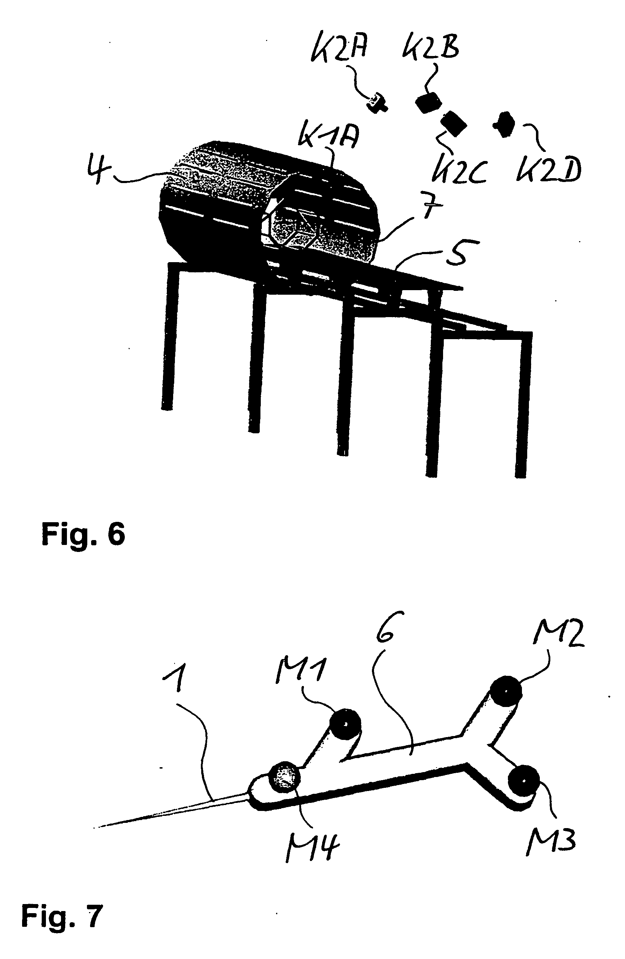

Fig. 6 zeigt eine perspektivische Darstellung des Kernspin- oder Computer-Tomographen von Fig. 1. Das System zur Gewinnung der Oberflächeninformation in der Modalität umfaßt gemäß dieser Variante der Erfindung vier erste optische Meßeinrichtungen, nämlich vier CMOS-Kameras, von welchen in Fig. 6 nur zwei dargestellt sind (K1A,K1B). Das CMOS-Kamerasystem K1A,K1B in der Modalität 4 dient zur Aufnahme der Körperoberfläche 3 und deren Bewegung. Diese Daten werden zeitlich synchronisiert mit den Volumeninformationen des Patienten aufgezeichnet. Eine vergleichbare Anordnung von vier zweiten optischen Meßeinrichtungen, nämlich vier weiteren CMOS-Kameras K2A,K2B,K2C,K2D, befindet sich über dem OPTisch außerhalb der Modalität. Die vier weiteren CMOS-Kameras K2A,K2B,K2C,K2D liefern die zu vergleichende Oberflächeninformation des Eingriffbereiches des Patienten in der Operationslage.

Der prinzipielle Aufbau des Systems geht aus Fig. 6 hervor. Die Röhre stellt in Fig. 6 den Aufnahmebereich der Modalität 4 dar, innerhalb der die ersten optischen Meßeinrichtungen angebracht sind. In Fig. 6 befindet sich innerhalb der Röhre 4 ein Kalibriergestell 7 zur Registrierung des Koordinatensystems der Modalität und des Multikamerasystems, welches die ersten optischen Meßeinrichtungen bildet.

Fig. 7 zeigt einen Trägerkörper 6, welcher an dem medizinischen Instrument 1 starr angeordnet ist und zur Lokalisierung einer Eingriffsposition dient. Der Trägerkörper 6 ist in der hier gezeigten Ausführungsform ein spezieller Handgriff, der mit vier farbigen Markierungen M1,M2,M3,M4 versehen ist. Bei bekannter Position der Markierungen auf dem Trägerkörper 6 kann mittels bekannter Bildverarbeitungsverfahren die Drehlage des medizinischen Instruments 1 im Raum und relativ zur Patientenoberfläche bestimmt werden.

Auf dem Oberflächenabschnitt 3 des Patienten werden gemäß dieser Variante der Erfindung die Referenzmarken R1,R2,R3 in Form eines regelmäßigen oder unregelmäßigen Gitters angebracht. Die Zahl der Refrenzmarken beträgt z.B. 25. Dieses kann über ein großes Pflaster geschehen, auf dem die Refrenzmarken R1,R2,R3 aufgedruckt sind. Während der Aufnahme von Volumendaten des Patienten werden die von den vier ersten optischen Meßeinrichtungen aufgenommenen Bilder zeitsynchron abgespeichert. Es entsteht so ein Bewegungsbild der Patientenoberfläche während der Aufnahme. Die Refrenzmarken erleichtern die Identifikation von Oberflächenmerkmalen für die Bewegungsverfolgung. Der Zusammenhang zwischen Oberflächenbewegung und Volumeninformationen kann Offline ermittelt werden. Die Synchronisation der Volumeninformation mit den Oberflächeninformation erfolgt über die Herstellung des zeitlichen Bezuges zwischen Modalität und externem Bildverarbeitungssystem. Die Positionsbestimmung der Marker erfolgt über den Ansatz eines Stereo-Vision-Systems. Als Ergebnis der Berechnung existiert bei dieser Variante der Erfindung in idealer Weise für jeden Zeitpunkt der Patientenbewegung eine Oberflächendatensatz, zu dem wiederum ein Volumendatensatz zugeordnet ist.

In der Operationssituation nehmen weitere vier CMOS-Kameras, nämlich die zweiten optischen Meßeinrichtungen K2A,K2B,K2C,K2D, die Oberflächensituation des Patienten kontinuierlich auf. Ein Vergleich der aktuellen Refrenzmarkenpositionen mit den in der Modalität 4 gewonnen Koordinatenmustern liefert eine Verbindung zu der entsprechenden 3D-Information, die dann visualisiert werden kann. Durch Einbeziehung eines mit Markierungen M1,M2,M3,M4 versehenden medizinischen Instruments 1 kann sich der Operateur vom System zusätzlich spezielle Schichtbilder entsprechend der Drehlage des medizinischen Instrumenzt 1 anzeigen lassen, wodurch eine Orientierung im Operationsgebiet erleichtert wird.

Zur schnellen Visualisierung der aktuellen Operationssituation können vorteilhaft moderne Grafikkarten eingesetzt werden, die mit hochintegrierten Grafikbeschleunigern versehen sind. Diese hochspezialisierten Chips verfügen über Fähigkeiten, die z.B. durch hardwarenahe Programmierung bzw. Programmierung auf Systemebene in vollem Umfang ausgenutzt werden können. Die Darstellung kann sowohl auf konventionellen Displays oder auf speziellen Displays (Head-Mounted-Device o.ä.) erfolgen.

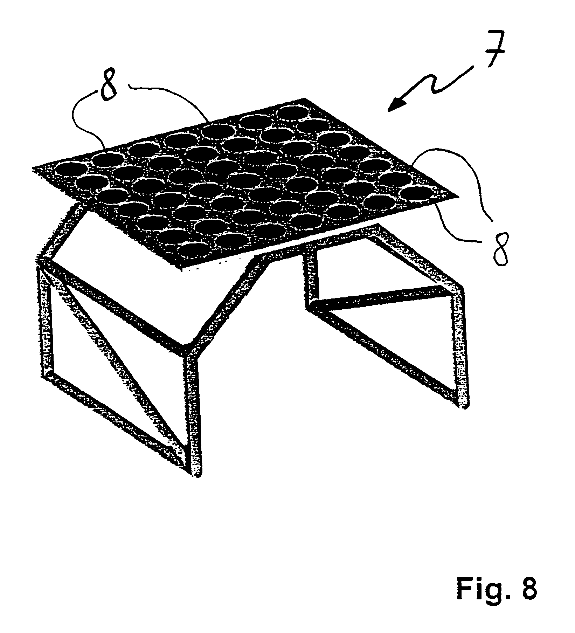

Fig. 8 zeigt eine perspektivische Darstellung des Kalibriergestells 7 von Fig. 6, welches mit Zielmarkierungen 8 versehen ist und gemäß einer Variante des erfindungsgemäßen Verfahrens zur Kalibrierung an einer definierten Stelle in dem Kernspin- oder Computer-Tomographen 4 aufgestellt wird. Das Kalibriergestell 7 stellt die Zielmarken 8 zur Bestimmung der gegenseitigen Relativlage der ersten optischen Meßeinrichtungen und der Relativlage derselben gegenüber dem Kernspin- oder Computer-Tomographen 4 bereit.

### Gewerbliche Anwendbarkeit:

Die Erfindung ist gewerblich anwendbar z.B. im Bereich der Medizintechnik, der Chirurgie, der Diagnostik und der Therapie von Erkrankungen durch lokale Injektion von Wirkstoffen.
Leitfigur ist Fig. 1.

### Liste der Bezugszeichen:

- 1: medizinisches Instrument
- 2: Körper
- 3: Oberflächenabschnitt
- 4: Kernspin- oder Computer-Tomograph
- 5: Tisch
- 6: Trägerkörper
- 7: Kalibriergestell
- 8: Zielmarkierungen auf 7
- K1A,K1B: erste optische Meßeinrichtungen
- K2A,K2B,K2C,K2D: zweite optische Meßeinrichtungen
- K3A,K3B: dritte optische Meßeinrichtungen
- K4A,K4B: vierte optische Meßeinrichtungen
- M1,M2,M3,M4: Markierungen
- P1,P2,P3: Oberflächenpunkte
- R1,R2,R3: Referenzmarken
- R1',R2',R3': erste Aufpunkte
- R1'',R2'',R3'': zweite Aufpunkte
- R1''',R2''',R3''': dritte Aufpunkte
- RF: Referenfläche
- RF'*,RF'',RF''': erste, zweite, dritte Aufpunkt-Referenzfläche
- S: medizinisch relevante Struktur
- ϕ1,ϕ2,ϕ3: erste, zweite, dritte Phase

## Patentansprüche

1. Verfahren zur Bestimmung der Orientierung und der Relativposition eines medizinischen Instruments (1) gegenüber einer medizinisch relevanten Struktur (S), z.B. Knochen oder Tumor, im Körper (2) eines atmenden Menschen oder atmenden Tieres, dessen Oberfläche einen Oberflächenabschnitt (3) umfaßt, welcher sich aufgrund der Atmung gegenüber der Struktur (S) zumindest zeitweise bewegt, zum Zweck eines medizinischen Eingriffs, wobei das medizinische Instrument (1) mit mindestens zwei voneinander beabstandeten, an demselben starr angeordneten Markierungen (M1, M2, M3, M4) versehen ist, deren Relativposition gegenüber dem medizinischen Instrument (1) jeweils bekannt ist, umfassend folgende Schritte:
a) in wenigstens einer ersten Phase (ϕ1) des Atemzyklusses des Menschen oder Tieres wird mindestens ein die Struktur (S) enthaltender Teil des Körpers (2) einer Kernspin- oder Computer-Tomographie unterzogen, mittels welcher die Struktur (S) sowie eine den Oberflächenabschnitt (3) aufspannende Vielzahl von Oberflächenpunkten (P1,P2,P3) erfaßt und die in der ersten Phase (ϕ1) vorliegende gegenseitige Relativposition der Oberflächenpunkte (P1,P2,P3) sowie die Relativposition der Struktur (S) gegenüber mindestens einem der Oberflächenpunkte (P1,P2,P3) bestimmt werden,
b) auf dem Oberflächenabschnitt (3) wird ein Satz von mindestens drei nicht kollinearen Referenzmarken (Rl,R2,R3), welche eine ebene oder nicht ebene Referenzfläche (RF) aufspannen, die an der atmungsbedingten Bewegung des Oberflächenabschnitts (3) teilnimmt, ausgewählt, wobei der Ort jeder Referenzmarke (R1,R2,R3)
- in der ersten Phase (ϕ1) des Atemzyklusses durch je einen der Referenzmarke (R1,R2,R3) ein-eindeutig zugeordneten ersten Aufpunkt (R1',R2',R3'), welche eine ebene oder nicht ebene erste Aufpunkt-Referenzfläche (RF') aufspannen,
- in einer zweiten Phase (ϕ2) des Atemzyklusses durch je einen der Referenzmarke (R1,R2,R3) ein-eindeutig zugeordneten zweiten Aufpunkt (R1",R2",R3"), welche eine ebene oder nicht ebene zweite Aufpunkt-Referenzfläche (RF") aufspannen, und
- in einer dritten Phase (ϕ3) des Atemzyklusses durch je einen der Referenzmarke (R1,R2,R3) ein-eindeutig zugeordneten dritten Aufpunkt (R1"',R2"',R3"'), welche eine ebene oder nicht ebene zweite Aufpunkt-Referenzfläche (RF"') aufspannen,
gegeben ist,
c) mittels mindestens zwei voneinander beabstandeten ersten optischen Meßeinrichtungen (K1A,K1B) wird die Relativposition von mindestens einem der ersten Aufpunkte (R1',R2',R3') gegenüber mindestens einem der zweiten Aufpunkte (R1",R2",R3") stereographisch oder trigonometrisch oder durch Bündelausgleich bestimmt,
d) mittels mindestens zwei voneinander beabstandeten zweiten optischen Meßeinrichtungen (K2A,K2B,K2C,K2D) werden die gegenseitigen Relativpositionen von mindestens dreien der zweiten Aufpunkte (R1", R2", R3") stereographisch oder trigonometrisch oder durch Bündelausgleich bestimmt,
e) die Relativposition von mindestens einem der zweiten Aufpunkte (R1", R2", R3") gegenüber mindestens einem der Oberflächenpunkte (P1, P2, P3) wird aus dem Ergebnis der Schritts c) und durch Ermittlung der Position der ersten oder der zweiten Aufpunkt-Referenzfläche (RF', RF") auf dem Oberflächenabschnitt (3) durch Matching zwischen der Orientierung und Gestalt der ersten bzw. zweiten Aufpunkt-Referenzfläche (RF',RF") und der Orientierung und der Gestalt des Oberflächenabschnitts (3) bestimmt,
f) mittels mindestens zwei voneinander beabstandeten dritten optischen Meßeinrichtungen (K3A, K3B) wird die gegenseitige Relativposition von mindestens einem der dritten Aufpunkte (R1"', R2"', R3"') gegenüber mindestens einem der ersten oder zweiten Aufpunkte (R1', R2', R3', R1", R2", R3") stereographisch oder trigonometrisch oder durch Bündelausgleich bestimmt,
g) mittels mindestens zwei voneinander beabstandeten vierten optischen Meßeinrichtungen (K4A,K4B) wird die Relativposition von mindestens zwei der Markierungen (M1,M2,M3,M4) des medizinischen Instruments (1) gegenüber jeweils mindestens einem der dritten Aufpunkte (R1'"R2'",R3'") stereographisch oder trigonometrisch oder durch Bündelausgleich bestimmt, und
h) aus den so gewonnenen Ergebnissen werden für die dritte Phase (ϕ3) des Atemzyklusses die Orientierung und die Relativposition des medizinischen Instruments (1) gegenüber der Struktur (S) bestimmt.

2. Verfahren nach Anspruch 1, **dadurch gekennzeichnet,**
**daß** die erste und die zweite Phase (ϕ1,ϕ2) bezüglich ihrer Lage im Atemzyklus zusammenfallen, so daß die ersten Aufpunkte (R1',R2',R3') mit den zweiten Aufpunkten (R1",R2",R3") identisch sind, und der Verfahrensschritt c) entfällt.

3. Verfahren nach Anspruch 1, **dadurch gekennzeichnet,**
**daß** die erste und die dritte Phase (ϕ1,ϕ3) bezüglich ihrer Lage im Atemzyklus zusammenfallen, so daß die ersten Aufpunkte (R1',R2',R3') mit den dritten Aufpunkten (R1"',R2"',R3"') identisch sind, und der Verfahrensschritt f) entfällt.

4. Verfahren nach Anspruch 1, **dadurch gekennzeichnet,**
**daß** die ersten und/oder zweiten und/oder dritten und/oder vierten optischen Meßeinrichtungen (K1A,K1B,K2A,K2B,K2C,K2D,K3A,K3B,K4A,K4B) identisch sind.

5. Verfahren nach Anspruch 4, **dadurch gekennzeichnet,**
**daß** die Anzahl der ersten, zweiten, dritten und vierten optischen Meßeinrichtungen (K1A, K1B, K2A, K2B, K2C, K2D, K3A, K3B, K4A, K4B) jeweils gleich vier ist.

6. Verfahren nach einem der Ansprüche 1 bis 5, **dadurch gekennzeichnet,**
**daß** die ersten, zweiten, dritten und vierten optischen Meßeinrichtungen (K1A,K1B,K2A,K2B,K2C,K2D,K3A,K3B,K4A,K4B) jeweils Videokameras sind.

7. Verfahren nach Anspruch 1, **dadurch gekennzeichnet,**
**daß** im Atemzyklus des Menschen oder Tieres die erste Phase (ϕ1) jeweils dem Übergang vom Einatmen zum Ausatmen und die dritte Phase (ϕ3) jeweils dem Übergang vom Ausatmen zum Einatmen entspricht.

8. Verfahren nach einem der Ansprüche 1 bis 7, **dadurch gekennzeichnet,**
**daß** die Referenzmarken (R1, R2, R3) auf einer Trägerfläche, z.B. Pflaster, markiert sind, welche auf der Oberfläche des Körpers lösbar fixiert, z.B. aufgebklebt wird.

9. Verfahren nach Anspruch 1, **dadurch gekennzeichnet,**
**daß** der Verfahrensschritt a) für eine Mehrzahl oder Vielzahl verschiedener, in ihrer Phasenlage bezüglich des Atemzyklus voneinander beabstandeter erster Phasen (ϕ1) wiederholt wird, so daß für jede der ersten Phasen (ϕ1) ein eigener, zu dieser ersten Phase (ϕ1) gehörender Satz von gegenseitigen Relativpositionen der Oberflächenpunkte (P1,P2,P3) sowie eine zu dieser ersten Phase (ϕ1) gehörende Relativposition der Struktur (S) gegenüber mindestens einem der Oberflächenpunkte (P1,P2,P3) bestimmt werden.

10. Verfahren nach Anspruch 1 oder 9, **dadurch gekennzeichnet,**
**daß** der Verfahrensschritt d) für eine Mehrzahl oder Vielzahl verschiedener, in ihrer Phasenlage bezüglich des Atemzyklus voneinander beabstandeter zweiter Phasen (ϕ2) wiederholt wird, so daß für jede der zweiten Phasen (ϕ2) ein eigener, zu dieser zweiten Phase (ϕ2) gehörender Satz von gegenseitigen Relativpositionen der zweiten Aufpunkte (R1", R2", R3") bestimmt wird.

11. Verfahren nach den Ansprüchen 2, 9 und 10, **dadurch gekennzeichnet,**
**daß** die Anzahl der ersten Phasen (ϕ1) und die Anzahl der zweiten Phasen (ϕ2) identisch sind, so daß jeder der ersten Phasen (ϕ1) genau eine der zweiten Phasen (ϕ2) zugeordnet ist, und die Phasenlage jeder der ersten Phasen (ϕ1) mit der Phasenlage der ihr zugeordneten zweiten Phase (ϕ2) übereinstimmt.

12. Verfahren nach Anspruch 11, **dadurch gekennzeichnet,**
**daß** jede Durchführung des Verfahrensschrittes a) mit einer Durchführung des Verfahrensschrittes d) zeitlich zusammenfällt, so daß die Durchführung der Verfahrensschritte a) mit der Durchführung des Verfahrensschrittes d) jeweils synchron erfolgt.

13. Verfahren nach Anspruch 12, **dadurch gekennzeichnet,**
**daß** alle Wiederholungen des Verfahrensschritts a) und des Verfahrensschrittes d) innerhalb eines einzigen, nämlich eines ersten Atemzyklusses erfolgen.

14. Verfahren nach Anspruch 1 oder 12, **dadurch gekennzeichnet,**
**daß** die Verfahrensschritte f) bis h) für eine Mehrzahl oder Vielzahl verschiedener, in ihrer Phasenlage bezüglich des Atemzyklus voneinander beabstandeter dritter Phasen (ϕ3) wiederholt werden, so daß für jede der dritten Phasen (ϕ3) die Orientierung und die Relativposition des medizinischen Instruments (1) gegenüber der Struktur (S) neu bestimmt werden.

15. Verfahren nach den Ansprüchen 3, 9,10,11 und 14, **dadurch gekennzeichnet,**
**daß** die Anzahl der zweiten Phasen (ϕ2) und die Anzahl der dritten Phasen (ϕ3) identisch sind, so daß jeder der dritten Phasen (ϕ3) eine der zweiten Phasen (ϕ2) eindeutig zugeordnet ist, und die Phasenlage jeder der dritten Phasen (ϕ3) mit der Phasenlage der ihr zugeordneten zweite Phasen (ϕ2) übereinstimmt, wobei für jede der dritten Phasen (ϕ3) die Orientierung und die Relativposition des medizinischen Instruments (1) gegenüber der Struktur (S) unter Verwendung
- desjenigen Satzes von gegenseitigen Relativpositionen der zweiten Aufpunkte (R1", R2", R3"), welcher zu der dieser dritten Phase (ϕ3) zugeordneten zweiten Phase (ϕ2) gehört,
- desjenigen Satzes von gegenseitigen Relativpositionen der Oberflächenpunkte (P1, P2, P3), welcher zu der dieser zweiten Phase (ϕ2) zugeordneten ersten Phase (ϕ1) gehört, und
- derjenigen Relativposition der Struktur (S) gegenüber mindestens einem der Oberflächenpunkte (P1, P2, P3), welche zu der dieser zweiten Phase (ϕ2) zugeordneten ersten Phase (ϕ1) gehört,
neu bestimmt wird.

16. Verfahren nach Anspruch 15, **dadurch gekennzeichnet,**
**daß** die Verfahrensschritte f) bis h) erst nach Ende des ersten Atemzyklusses durchgeführt werden.

17. Verfahren nach Anspruch 1 oder 16, **dadurch gekennzeichnet,**
**daß** die Verfahrensschritte f) bis h) über eine Mehrzahl von Atemzyklen hinweg fortlaufend wiederholt werden.

18. Verfahren nach einem der Ansprüche 14 bis 17, **dadurch gekennzeichnet,**
**daß** die Orientierung bzw. die Relativposition des medizinischen Instruments (1) gegenüber der Struktur (S) für eine beliebige vierte Phase des Atemzyklusses gewonnen werden, indem zwischen den für zwei verschiedene dritten Phasen (ϕ3) ermittelten Orientierungen des medizinischen Instruments (1) bzw. zwischen den für zwei verschiedene dritten Phasen (ϕ3) ermittelten Relativposition des medizinischen Instruments (1) gegenüber der Struktur (S) linear oder nichtlinear interpoliert wird.

19. Verfahren nach einem der Ansprüche 15 bis 18, **dadurch gekennzeichnet,**
**daß** die jeweils zuletzt bestimmte Orientierung und Relativposition des medizinischen Instruments (1) gegenüber der Struktur (S) auf mindestens einem Monitor graphisch dargestellt werden.

20. Verfahren nach Anspruch 19, **dadurch gekennzeichnet,**
**daß** auf dem Monitor bzw. den Monitoren zusätzlich die jeweils zuletzt bestimmte Relativposition der Referenzmarken (R1, R2, R3) gegenüber der Struktur (S) in Echtzeit dargestellt wird.

21. Verfahren nach Anspruch 19 oder 20, **dadurch gekennzeichnet,**
**daß** die Darsatellung auf dem Monitor bzw. den Monitoren in Echtzeit erfolgt.

22. Verfahren nach einem der Ansprüche 1. bis 21, **dadurch gekennzeichnet,**
**daß** sich die ersten optischen Meßeinrichtungen (K1A, K1B) innerhalb des Kernspin- oder Computer-Tomographen (4) befinden.

23. Verfahren nach einem der Ansprüche 1 bis 22, **dadurch gekennzeichnet,**
**daß** die Markierungen (M1, M2, M3, M4) starr an einem Trägerkörper (6) angeordnet sind, welcher seinerseits starr und lösbar mit dem medizinischen Instrument (1) verbunden und nacheinander an verschiedenen medizinischen Instrumenten einsetzbar ist.

## Claims

1. A method to determine the orientation and the relative position of a medical instrument (1) with respect to a medically relevant structure (S) such as, for instance, a bone or tumor in the body (2) of a breathing person or breathing animal whose surface encompasses a surface section (3) which, at least at times, moves relative to the structure (S) because of the breathing, said method being for purposes of a medical procedure, whereby the medical instrument (1) is provided with at least two markings (M1, M2, M3, M4) rigidly arranged on it at a distance from each other, whose relative position with respect to the medical instrument (1) is known in each case, said method comprising the following steps:
a) in at least a first phase (ϕ1) of the breathing cycle of the person or animal, at least a part of the body (2) containing the structure (S) undergoes nuclear magnetic resonance tomography or computer tomography by means of which the structure (S) as well as a plurality of surface points (P1, P2, P3) encompassing the surface section (3) are detected and the reciprocal relative position of the surface points (P1, P2, P3) present in the first phase (ϕ1) as well as the relative position of the structure (S) with respect to at least one of the surface points (P1, P2, P3) are determined,
b) a set is selected consisting of at least three non-collinear reference marks (R1, R2, R3) encompassing a planar or non-planar reference surface (RF) that participates in the movement of the surface section (3) caused by the breathing, whereby
- in the first phase (ϕ1) of the breathing cycle, the location of each reference mark (R1, R2, R3) is defined by a first space point (R1', R2', R3') that is unambiguously associated with one of the reference marks (R1, R2, R3) encompassing a planar or non-planar first space point reference surface (RF'),
- in a second phase (ϕ2) of the breathing cycle, the location of each reference mark (R1, R2, R3) is defined by a second space point (R1", R2", R3") that is unambiguously associated with one of the reference marks (R1, R2, R3) encompassing a planar or non-planar second space point reference surface (RF"), and
- in a third phase (ϕ3) of the breathing cycle, the location of each reference mark (R1, R2, R3) is defined by a third space point (R1"', R2"', R3"') that is unambiguously associated with one of the reference marks (R1, R2, R3) encompassing a planar or non-planar third space point reference surface (RF'"),
c) by means of at least two first optical measuring devices (K1A, K1B) arranged at a distance from each other, the relative position of at least one of the first space points (R1', R2', R3') with respect to at least one of the second space points (R1", R2", R3") is determined stereographically or trigonometrically or by bundle balancing,
d) by means of at least two second optical measuring devices (K2A, K2B, K2C, K2D) arranged at a distance from each other, the reciprocal relative positions of at least three of the second space points (R1", R2", R3") are determined stereographically or trigonometrically or by bundle balancing,
e) the relative position of at least one of the second space points (R1", R2", R3") with respect to at least one of the surface points (P1, P2, P3) is determined from the result of step c) and by ascertaining the position of the first or second space point reference surface (RF', RF") on the surface section (3) through matching between the orientation and shape of the first or second space point reference surface (RF', RF") and the orientation and shape of the surface section (3),
f) by means of at least two third optical measuring devices (K3A, K2B) arranged at a distance from each other, the reciprocal relative position of at least one of the third space points (R1'", R2'", R3'") with respect to at least one of the first or second space points (R1', R2', R3', R1", R2", R3") is determined stereographically or trigonometrically or by bundle balancing,
g) by means of at least two fourth optical measuring devices (K4A, K4B) arranged at a distance from each other, the relative position of at least two of the markings (M1, M2, M3, M4) of the medical instrument (1) with respect to at least one of the third space points (R1"', R2"', R3"') is determined stereographically or trigonometrically or by bundle balancing,
h) from the results thus obtained, the orientation and the relative position of the medical instrument (1) with respect to the structure (S) are determined for the third phase (ϕ3) of the breathing cycle.

2. The method according to Claim 1, **characterized in that**
the first and the second phases (ϕ1, ϕ2) coincide in terms of their positioning in the breathing cycle, so that the first space points (R1', R2', R3') are identical to the second space points (R1", R2", R3") and method step c) is dispensed with.

3. The method according to Claim 1, **characterized in that**
the first and third phases (cp1, ϕ3) coincide in terms of their positioning in the breathing cycle, so that the first space points (R1', R2', R3') are identical to the third space points (R1"', R2"', R3"') and method step f) is dispensed with.

4. The method according to Claim 1, **characterized in that** the first and/or second and/or third and/or fourth optical measuring devices (K1A, K1B, K2A, K2B, K2C, K2D, K3A, K3B, K4A, K4B) are identical.

5. The method according to Claim 4, **characterized in that**
the number of first, second, third and fourth optical measuring devices (K1A, K1B, K2A, K2B, K2C, K2D, K3A, K3B, K4A, K4B) is four in each case.

6. The method according to any of Claims 1 to 5, **characterized in that**
the first, second, third and fourth optical measuring devices (K1A, K1B, K2A, K2B, K2C, K2D, K3A, K3B, K4A, K4B) are all video cameras.

7. The method according to Claim 1, **characterized in that**,
in the breathing cycle of the person or animal, the first phase (ϕ1) corresponds each time to the transition from inhalation to exhalation and the third phase (ϕ3) corresponds each time to the transition from exhalation to inhalation.

8. The method according to any of Claims 1 to 7, **characterized in that**
the reference marks (R1, R2, R3) are marked on a support surface, for instance, an adhesive tape, that is removably attached, for example, glued, onto the surface of the body.

9. The method according to Claim 1, **characterized in that**
method step a) is repeated for several or many different first phases (ϕ1) that are at a distance from each other in terms of their phase in the breathing cycle, so that, for each of the first phases (ϕ1), a set of reciprocal relative positions of the surface points (P1, -P2, P3) - said set belonging to this first phase (ϕ1) - as well as a relative position of the structure (S) - said position belonging to this first phase (ϕ1) - are determined with respect to at least one of the surface points (P1, P2, P3).

10. The method according to Claim 1 or 9, **characterized in that**
method step d) is repeated for several or many different second phases (ϕ2) that are at a distance from each other in terms of their phase in the breathing cycle, so that, for each of the second phases (ϕ2), a set of reciprocal relative positions of the second space points (R1", R2", R3") - said set belonging to this second phase (ϕ2) - is determined.

11. The method according to Claims 2, 9 and 10, **characterized in that**
the number of first phases (ϕ1) and the number of second phases (ϕ2) is identical, so that each of the first phases (ϕ1) is precisely associated with one of the second phases (ϕ2), and the phase positioning of each of the first phases (ϕ1) coincides with the phase positioning of the second phase (ϕ2) associated with them.

12. The method according to Claim 11, **characterized in that**
the time of the execution of method step a) coincides with the time of the execution of method step d), so that the execution of method steps a) always takes place synchronously with the execution of method step d).

13. The method according to Claim 12, **characterized in that**
all of the repetitions of method step a) and of method step d) take place within one single breathing cycle, namely, the first one.

14. The method according to Claim 1 or 12, **characterized in that**
method steps f) through h) are repeated for several or many different third phases (ϕ3) that are at a distance from each other in terms of their phase in the breathing cycle, so that, for each of the third phases (ϕ3), the orientation and the relative position of the medical instrument (1) with respect to the structure (S) are determined anew.

15. The method according to Claims 3, 9, 10, 11 and 14, **characterized in that** the number of second phases (ϕ2) and the number of third phases (ϕ3) is identical, so that each of the third phases (ϕ3) is unambiguously associated with one of the second phases (ϕ2), and the phase positioning of each of the third phases (ϕ3) coincides with the phase positioning of the second phases (ϕ2) associated with them, whereby for each of the third phases (ϕ3), the orientation and the relative position of the medical instrument (1) with respect to the structure (S) are determined anew employing
• the set of reciprocal relative positions of the second space points (R1", R2", R3") that belongs to the second phase (ϕ2) associated with this third phase (ϕ3),
• the set of reciprocal relative positions of the surface points (P1, P2, P3) that belongs to the first phase (ϕ1) associated with this second phase (ϕ2), and
• the relative position of the structure (S) with respect to at least one of the surface points (P1, P2, P3) that belongs to the first phase (ϕ1) associated with this second phase (ϕ2).

16. The method according to Claim 15, **characterized in that**
the method steps f) through h) are only carried out after the end of the first breathing cycle.

17. The method according to Claim 1 or 16, **characterized in that**
method steps f) through h) are repeated continually over numerous breathing cycles.

18. The method according to any of Claims 14 to 17, **characterized in that** the orientation or the relative position of the medical instrument (1) with respect to the structure (S) is acquired for any desired fourth phase of the breathing cycle **in that** a linear or non-linear interpolation is carried out among the orientations of the medical instrument (1) ascertained for two different third phases (ϕ3) or else among the relative positions of the medical instrument (1) with respect to the structure (S) ascertained for two different third phases (ϕ3).

19. The method according to any of Claims 15 to 18, **characterized in that**, each time, the most recently ascertained orientation and relative position of the medical instrument (1) with respect to the structure (S) are displayed in graphic form on at least one monitor.

20. The method according to Claim 19, **characterized in that**,
each time, the most recently ascertained relative position of the reference marks (R1, R2, R3) with respect to the structure (S) is displayed in real time on the monitor or monitors.

21. The method according to Claim 19 or 20, **characterized in that**
the display on the monitor or monitors is shown in real time.

22. The method according to any of Claims 1 to 21, **characterized in that**
the first optical measuring devices (K1A, K1 B) are located inside the nuclear magnetic resonance tomograph or computer tomograph (4).

23. The method according to any of Claims 1 to 22, **characterized in that**
the markings (M1, M2, M3, M4) are rigidly arranged on a support element (6) which, in turn, is rigidly and removably connected to the medical instrument (1) and can be employed consecutively with various medical instruments.

## Revendications

1. Méthode pour déterminer l'orientation et la position relative d'un instrument médical (1) par rapport à une structure médicalement importante (S), telle que, par exemple, un os ou une tumeur dans le corps (2) d'un homme qui respire ou d'un animal qui respire, dont la surface comporte une section de surface (3) qui bouge tout du moins temporairement par rapport à la structure (S) en raison de la respiration, dans le but d'une intervention médicale, l'instrument médical (1) étant pourvu d'au moins deux marques-repères (M1, M2, M3, M4) espacées l'une de l'autre et disposées sur celui-ci de façon rigide, dont la position relative par rapport à l'instrument médical (1) est respectivement connue, comportant les étapes suivantes :
a) dans au moins une des phases (ϕ1) du cycle de respiration de l'homme ou de l'animal, au moins une partie du corps (2) contenant la structure (S) est soumise à une imagerie par résonance magnétique nucléaire ou à une tomographie par ordinateur au moyen de laquelle la structure (S) est saisie ainsi qu'une pluralité de points de surface (P1, P2, P3) déployant la section de surface (3), et la position relative mutuelle des points de surface (P1, P2, P3) donnée dans la première phase (ϕ1), ainsi que la position relative de la structure (S) par rapport à au moins un des points de surface (P1, P2, P3) sont déterminées,
b) sur la section de surface (3) est choisi un jeu d'au moins trois marques de référence (R1, R2, R3) non colinéaires qui déploient une surface de référence (RF) plane ou non plane, laquelle participe au mouvement de la section de surface (3) dû à la respiration, l'endroit de chaque marque de référence (R1, R2, R3) étant donné,
- dans la première phase (ϕ1) du cycle de respiration par un premier point spatial (R1', R2', R3') respectif assigné biunivoquement à la marque de référence (R1, R2, R3), lesquels points spatiaux déploient une première surface de référence des points spatiaux (RF") plane ou non plane, et
- dans une deuxième phase (ϕ2) du cycle de respiration par un deuxième point spatial (R1", R2", R3") respectif assigné biunivoquement à la marque de référence (R1, R2, R3), lesquels points spatiaux déploient une deuxième surface de référence de points spatiaux (RF"), plane ou non plane et
- dans une troisième phase (ϕ3) du cycle de respiration par un troisième point spatial (R1"', R2"', R3"') respectif assigné biunivoquement à la marque de référence (R1, R2, R3), lesquels points spatiaux déploient une troisième surface de référence des points spatiaux (RF"') plane ou non plane,
c) au moyen d'au moins deux premiers dispositifs de mesure optique (K1A, K1 B) espacés l'un de l'autre, la position relative d'au moins un des premiers points spatiaux (R1', R2', R3') par rapport à au moins un des deuxièmes points spatiaux (R1 ", R2", R3") est déterminée par stéréographie ou par trigonométrie ou par ajustement de faisceaux,
d) au moyen d'au moins deux des deuxièmes dispositifs de mesure optique (K2A, K2B, K2C, K2D) espacés l'un de l'autre, les positions relatives mutuelles d'au moins trois des deuxièmes points spatiaux (R1", R2", R3") sont déterminées par stéréographie ou par trigonométrie ou par ajustement de faisceaux,
e) la position relative d'au moins un des deuxièmes points spatiaux (R1", R2", R3") par rapport à au moins un des points de surface (P1, P2, P3) est déterminée à partir du résultat de l'étape c) et par la détermination de la position de la première ou de la deuxième surface de référence des points spatiaux (RF', RF") sur la section de surface (3) par comparaison entre l'orientation et la forme de la première ou de la deuxième surface de référence des points spatiaux (RF', RF") et l'orientation et la forme de la section de surface (3).
f) au moyen d'au moins deux troisièmes dispositifs de mesure (K3A, K3B) espacés l'un de l'autre, la position relative mutuelle d'au moins un des trois points spatiaux (R1"', R2"', R3"') par rapport à au moins un des premiers ou des deuxièmes points spatiaux (R1', R2', R3'), est déterminée par stéréographie ou par trigonométrie, ou par ajustement de faisceaux,
g) au moyen d'au moins deux quatrièmes dispositifs de mesure optique (K4A, K4B) espacés l'un de l'autre, la position relative d'au moins deux des marques-repères (M1, M2, M3, M4) de l'instrument médical (1) par rapport à au moins un des troisièmes points spatiaux (R1'", R2"', R3"') est déterminée par stéréographie ou par trigonométrie ou par ajustement de faisceaux, et
h) à partir des résultats ainsi obtenus, l'orientation et la position relative de l'instrument médical (1) par rapport à la structure (S) sont déterminées pour la troisième phase (ϕ3) du cycle de respiration.

2. Méthode selon la revendication 1, **caractérisée en ce que**
la première et la deuxième phase (ϕ1, ϕ2) coïncident en ce qui concerne leur position dans le cycle de respiration, de sorte que les premiers points spatiaux (R1', R2', R3') sont identiques aux deuxièmes points spatiaux (R1 ", R2", R3") et l'étape opérationnelle c) est supprimée.

3. Méthode selon la revendication 1, **caractérisée en ce que**
la première et la troisième phase (ϕ1, ϕ3) coïncident en ce qui concerne leur position dans le cycle de respiration, de sorte que les premiers points spatiaux (R1', R2', R3') sont identiques aux troisièmes points spatiaux (R1"', R2"', R3"') et l'étape opérationnelle f) est supprimée.

4. Méthode selon la revendication 1, **caractérisée en ce que**
les premiers et / ou deuxièmes et / ou troisièmes et / ou quatrièmes dispositifs de mesure optiques (K1A, K1B, K2A, K2B, K2C, K2D, K3A, K3B, K4A, K4B) sont identiques.

5. Méthode selon la revendication 4, **caractérisée en ce que**
le nombre des premiers, deuxièmes, troisièmes et quatrièmes dispositifs de mesure optique (K1A, K1B, K2A, K2B, K2C, K2D, K3A, K3B, K4A, K4B) est respectivement égal à quatre.

6. Méthode selon l'une des revendications 1 à 5, **caractérisée en ce que**
les premiers, deuxièmes, troisièmes et quatrièmes dispositifs de mesure optique (K1A, K1B, K2A, K2B, K2C, K2D, K3A, K3B, K4A, K4B) sont respectivement des caméras vidéo.

7. Méthode selon la revendication 1, **caractérisée en ce que**
dans le cycle de respiration de l'homme ou de l'animal, la première phase (ϕ1) correspond respectivement au passage de l'inspiration à l'expiration et la troisième phase (ϕ3) respectivement au passage de l'expiration à l'inspiration.

8. Méthode selon l'une des revendications 1 à 7, **caractérisée en ce que**
les marques de référence (R1, R2, R3) sont marquées sur un support, par exemple un sparadrap qui est maintenu sur la surface du corps de façon à pouvoir être enlevé, par collage, par exemple. ,

9. Méthode selon la revendication 1, **caractérisée en ce que**
l'étape opérationnelle a) est répétée pour la majeure partie ou la pluralité de diverses premières phases (ϕ1) espacées les unes des autres dans leur position de phase en ce qui concerne le cycle de respiration, de sorte que, pour chacune des premières phases (ϕ1), un propre jeu de positions relatives mutuelles des points de surface (P1, P2, P3) faisant partie de cette première phase (ϕ1), ainsi que la position relative de la structure (S) faisant partie de cette première phase (ϕ1), par rapport à au moins un des points de surface (P1, P2, P3) sont déterminés.

10. Méthode selon l'une des revendications 1 à 9, **caractérisée en ce que**
l'étape opérationnelle d) est répétée pour une majeure partie ou une pluralité de diverses deuxièmes phases (ϕ2) espacées les unes des autres dans leur position de phase en ce qui concerne le cycle de respiration, de sorte que, pour chacune des deuxièmes phases (ϕ2), un propre jeu de positions relatives mutuelles des deuxièmes points spatiaux mécaniques (R1", R2", R3") faisant partie de cette deuxième phase (ϕ2) est déterminé.

11. Méthode selon les revendications 2, 9 et 10, **caractérisée en ce que**
le nombre des premières phases (ϕ1) et le nombre des deuxièmes phases (ϕ2) est identique, de sorte qu'à chacune des premières phases (ϕ1) est assignée exactement une des deuxièmes phases (ϕ2), et la position de phase de chacune des premières phases (ϕ1) concorde avec la position de phase de la deuxième phase (ϕ2) qui lui est assignée.

12. Méthode selon la revendication 11, **caractérisée en ce que**
chaque exécution de l'étape opérationnelle a) coïncide dans le temps avec l'exécution de l'étape opérationnelle d), de sorte que l'exécution des étapes opérationnelles a) se produit respectivement de façon synchrone avec l'exécution de l'étape opérationnelle d).

13. Méthode selon la revendication 12, **caractérisée en ce que**
toutes les répétitions de l'étape opérationnelle a) et de l'étape opérationnelle d) se produisent à l'intérieur d'un seul cycle de respiration, à savoir le premier.

14. Méthode selon l'une des revendications 1 ou 12, **caractérisée en ce que**
les étapes opérationnelles f) à h) se répètent pour une majeure partie ou une pluralité de diverses troisièmes phases (ϕ3) espacées les unes des autres dans leur position de phase en ce qui concerne le cycle de respiration, de sorte que pour chacune des troisièmes phases (ϕ3), l'orientation et la position relative de l'instrument médical (1) par rapport à la structure (S) doit être à nouveau déterminée.

15. Méthode selon les revendications 3, 9, 10, 11 et 14, **caractérisée en ce que**
le nombre des deuxièmes phases (ϕ2) et le nombre des troisièmes phases (ϕ3) est identique, de sorte que à chacune des troisièmes phases (ϕ3) est assignée univoquement une des deuxièmes phases (ϕ2) et la position de phase de chacune des troisièmes phases (ϕ3) concorde avec la position de phase de la deuxième phase (ϕ2) qui lui est assignée, l'orientation et la position relative de l'instrument médical (1) par rapport à la structure (S) devant être à nouveau déterminée pour chacune des troisièmes phases (ϕ3) en ayant recours
- au jeu des positions relatives mutuelles des deuxièmes points spatiaux (R1", R2", R3"), lequel fait partie de la deuxième phase (ϕ2) assignée à cette troisième phase (ϕ3),
- au jeu de positions relatives mutuelles des points de surface (P1, P2, P3), lequel fait partie de la première phase (ϕ1) assignée à cette deuxième phase (ϕ2), et
- à la position relative de la structure (S) par rapport à au moins un des points de surface (P1, P2, P3), laquelle fait partie de la première phase (ϕ1) assignée à cette deuxième phase (ϕ2).

16. Méthode selon la revendication 15, **caractérisée en ce que**
les étapes opérationnelles f) à h) sont exécutées seulement à la fin du premier cycle de respiration.

17. Méthode selon l'une des revendications 1 ou 16, **caractérisée en ce que**
les étapes opérationnelles f) à h) se répètent de façon continue sur une pluralité de cycles de respiration.

18. Méthode selon l'une des revendications 14 à 17, **caractérisée en ce que**
l'orientation ou la position relative de l'instrument médical (1) par rapport à la structure (S) peut être obtenue pour une quatrième phase quelconque du cycle de respiration, dans la mesure où une interpolation linéaire ou non linéaire est faite entre les orientations de l'instrument médical (1) déterminées pour deux troisièmes phases (ϕ 3) différentes ou entre les positions relatives de l'instrument médical (1) par rapport à la structure (S) déterminée pour deux troisièmes phases (ϕ3) différentes.

19. Méthode selon l'une des revendications 15 à 18, **caractérisée en ce que**
l'orientation et la position relative de l'instrument médical (1) par rapport à la structure (S) déterminées respectivement en dernier sont représentées graphiquement sur au moins un moniteur.

20. Méthode selon la revendication 19, **caractérisée en ce que**
sur le moniteur ou les moniteurs est représentée de plus en temps réel la position relative des marques de référence (R1, R2, R3) par rapport à la structure (S) déterminées respectivement en dernier.

21. Méthode selon l'une des revendications 19 ou 20, **caractérisée en ce que**
la représentation sur le ou les moniteurs a lieu en temps réel.

22. Méthode selon l'une des revendications 1 à 21, **caractérisée en ce que**
les premiers dispositifs de mesure optique (K1A, K1 B) se trouvent à l'intérieur du tomographe à résonance magnétique nucléaire ou du tomographe par ordinateur (4).

23. Méthode selon l'une des revendications 1 à 22, **caractérisée en ce que**
les marques-repères (M1, M2, M3, M4) sont disposés de façon rigide sur un support (6) lequel est lié à son tour de façon rigide à l'instrument médical (1) dont il peut être détaché et utilisé tour à tour sur plusieurs instruments médicaux.
